# EUROPEAN PATENT APPLICATION

(11) **EP 4 685 152 A1**
(43) Date of publication of application: **28.01.2026**
(21) Application number: 24775120.9
(22) Date of filing: 13.03.2024
(51) Int. Cl.: C07K 7/06, C07K 16/28, A61K 47/42, A61K 49/00, A61K 51/08, A61P 35/00, A61K 38/00, G01N 33/574, A61K 39/00

(54) **PEPTIDE BINDING TO LAG-3 AND USE THEREOF**

(30) Priority: 20.03.2023 KR 20230035632; 11.03.2024 KR 20240033776
(71) Applicant: Kyungpook National University Industry-Academic Cooperation Foundation, Daegu 41566 (KR)
(72) Inventor: LEE, Byungheon, Daegu 42038 (KR); LEE, Seokmin, Busan 47792 (KR)
(74) Representative: Mewburn Ellis LLP
(86) International application number: PCT/KR2024/003211
(87) International publication number: WO 2024/196072

(57) **Abstract**

The present invention relates to peptides binding to LAG-3 and a use thereof in cancer immunotherapy and anticancer applications. The peptides of the present invention specifically bind to and inhibit LAG-3, thereby activating the function of immune cells against cancer cells and exhibiting anticancer effects. Using phage peptide display technology targeting cells with high expression of human LAG-3 protein, two peptides, LAG-3Pep-1 and LAG-3Pep-2, which were found to highly bind to the protein, were selected as the peptides of the present invention. These peptides were confirmed to bind to both human and mouse LAG-3 and inhibit its function. The peptides of the present invention showed effects similar to those of antibodies and were relatively stable in the bloodstream, indicating high potential as future cancer immunotherapeutics.

## Description

### Technical Field

The present disclosure relates to a peptide binding to LAG-3 and a use in cancer immunotherapy and anticancer treatment using the same.

### Background Art

Recently, selective monoclonal antibodies targeting immune checkpoint molecules have achieved unprecedented success in the treatment of cancers that occur in humans. These developments in cancer immunotherapy are setting a new milestone in the history of cancer treatment. Lymphocyte-activation gene-3 (LAG-3), one of immune checkpoint molecules, is being an attractive therapeutic target in several cancers, as it is primarily upregulated upon T cell activation and is also abundant in exhausted T cells commonly shown in tumor-infiltrating lymphocytes. According to panel studies on human and mouse tumor samples, it was revealed that LAG-3 is expressed at a high level in tumor-infiltrating lymphocytes as well as in lymphocytes in secondary immune organs. LAG-3 is expressed in various types of lymphocytes and dendritic cells.

Blocking of an interaction between LAG-3 and its ligand, MHC class II, has shown excellent antitumor responses and clinical efficacy in some patients. Blocking of LAG-3/MHC class II interactions maintains and reactivates functions of helper T cells and increases cytokine production. Antibodies that block the LAG-3/MHC class II pathway have derived long-lasting and favorable clinical outcomes in a significant proportion of patients with an extensive, highly refractory cancer. Therefore, there is a great medical need to develop highly effective, low-cost inhibitors against LAG-3/MHC class II.

Although a significant number of cancer patients have shown effectiveness in treatment with a blockade of key immune checkpoints such as CTLA-4, PD-1, and PD-L1, responsiveness is still barely observed in many patients. In addition, it is believed that even better anticancer immunotherapy can be achieved by co-administering a blocker of the interaction between LAG-3, another immune checkpoint, and its ligands, MHC class II and FGL-1, along with the immune checkpoint blocker.

### Disclosure of the Invention

### Technical Goals

The present disclosure relates to a peptide binding to LAG-3 and a use thereof and, more specifically, the present disclosure provides a peptide specifically binding to LAG-3 consisting of an amino acid sequence represented by SEQ ID NO: 1 or SEQ ID NO: 2, a drug delivery composition including the peptide as an active ingredient, a pharmaceutical composition for preventing or treating a cancer and a composition for imaging T cells infiltrated into a cancer tissue; and a pharmaceutical composition for preventing or treating a cancer including the peptide and a PD-L1 antibody as active ingredients.

### Technical Solutions

To address the above object, the present disclosure provides a peptide that specifically binds to LAG-3, consisting of an amino acid sequence represented by SEQ ID NO: 1 or SEQ ID NO: 2.

In addition, the present disclosure provides a polynucleotide encoding the peptide, a recombinant vector including the polynucleotide, and a transformant which is transformed with the recombinant vector and isolated.

In addition, the present disclosure provides a drug delivery composition including the peptide as an active ingredient.

In addition, the present disclosure provides a pharmaceutical composition for preventing or treating a cancer, including the peptide as an active ingredient.

In addition, the present disclosure provides a pharmaceutical composition for preventing or treating a cancer, including the peptide and a PD-L1 antibody as active ingredients.

In addition, the present disclosure provides a composition for imaging T cells infiltrated into a cancer tissue, including the peptide as an active ingredient.

### Advantageous Effects

The present disclosure relates to a peptide binding to LAG-3 and a use in cancer immunotherapy and anticancer using the same, wherein the peptide of the present disclosure exhibits an anticancer effect by specifically binding to LAG-3 to inhibit the same, thereby activating a function of immune cells against cancer cells. The peptide of the present disclosure was obtained via a phage peptide display technology by targeting a cell in which a human LAG-3 protein is highly expressed to select two types of peptides (LAG3Pep-1 and LAG3Pep-2) that well bind to the cell, and it was shown that they bind to human and mouse LAG-3 and inhibit its function. The peptide of the present disclosure exhibited effects similar to that of antibodies with a high potential as a future cancer immunotherapeutic agent. Brief Description of Drawings

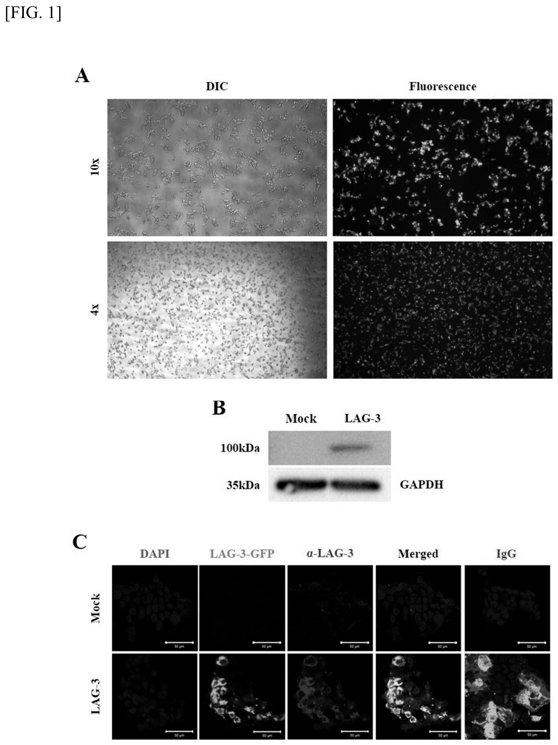

FIG. 1 shows expression of LAG-3 in HEK-293T cells which are transfected with plasmid DNA expressing LAG-3. (a) Images of expression of green fluorescent protein (GFP) co-expressed with LAG-3 48 hours after transiently transfecting LAG-3 plasmid DNA into HEK-293T cells, taken with DIC microscopy and fluorescence microscopy (10x and 4x). (b) A result of immunoblot analysis obtained by extracting proteins from cells 48 hours after transfection to determine a level of LAG-3 expression. (c) Images of expression levels of LAG-3 (red) and GFP (green), taken 48 hours after staining cells with LAG-3 antibody in cells expressing or not expressing LAG-3 with an immunofluorescence confocal microscope (Scale bar: 30 µm).

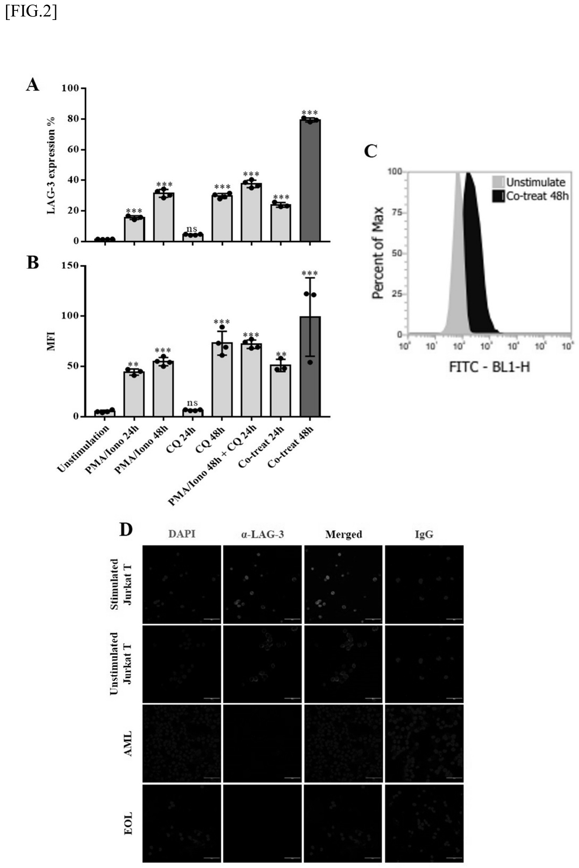

FIG. 2 shows expression levels of LAG-3 in a Jurkat T cell line. (a-b) Results obtained by measuring the percentage of LAG-3 expression and mean fluorescent intensity (MFI) via flow cytometry using LAG-3 antibody in Jurkat T cells stimulated in various ways using phorbol 12-myristate 13-acetate (PMA) (50 ng/ml), ionomycin (1 µg/ml), and chloroquine (CQ, 100 µM). *, P< 0.05; **, P< 0.01; ***, P< 0.001. (c) A result of flow cytometry shown in histogram for LAG-3 expression in stimulated Jurkat T cells. (d) A result of immunofluorescence analysis on an expression level of LAG-3 in stimulated and unstimulated Jurkat T cell lines using a confocal microscope. AML and EOL cells were used as control groups (Scale bar: 30 µm).

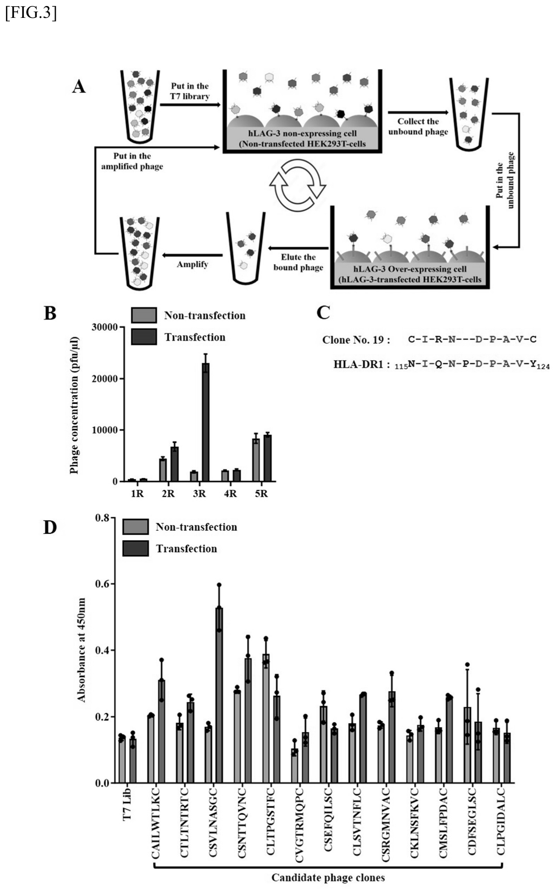

FIG. 3 shows bio-panning results for discovering LAG-3 binding peptides. (a) A schematic diagram of a bio-panning process. (b) The phage titer (pfu) recovered in each round of bio-panning performed five times in HEK-293T transfected and untransfected cells. (c) A result of searching in the NCBI-protein BLAST database, showing that a candidate peptide (named LAG-3Pep-1) has an amino acid sequence similar to that of HLA-DR1 (a subtype of human MHC class II), which is a ligand for LAG-3. (d) A result obtained by selecting 13 phage clones from HEK-293T transfected cells to analyze the binding of the clones to HEK-293T cells via phage binding ELISA. Optical density (OD) was measured at a wavelength of 450 nm.

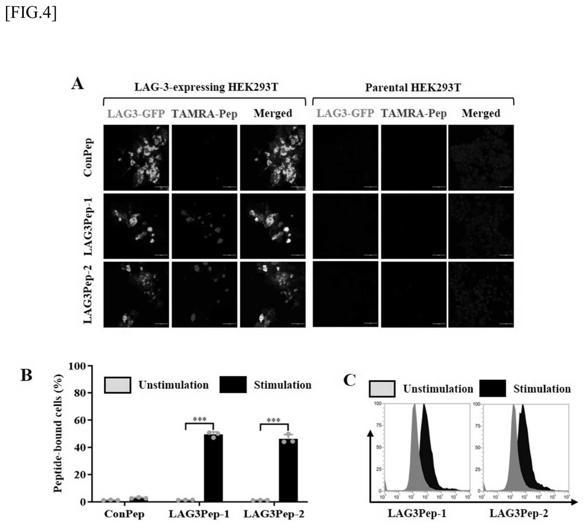

FIG. 4 shows a degree of binding of LAG-3 binding peptides (LAG3Pep-1, LAG3Pep-2) to cells. (a) Results of analyzing peptide binding to HEK-293T cells transfected or untransfected with a LAG-3 plasmid vector using immunofluorescence microscopy (Scale bar: 30 µm). (b) A result of determining a binding percentage of LAG-3 binding peptides in Jurkat T cells stimulated or unstimulated with PMA (50 ng/ml), ionomycin (1 µg/ml), and CQ (100 µM) by flow cytometry. *, P< 0.05; **, P< 0.01; ***, P< 0.001. (c) Results of flow cytometry, shown in a histogram for peptide binding.

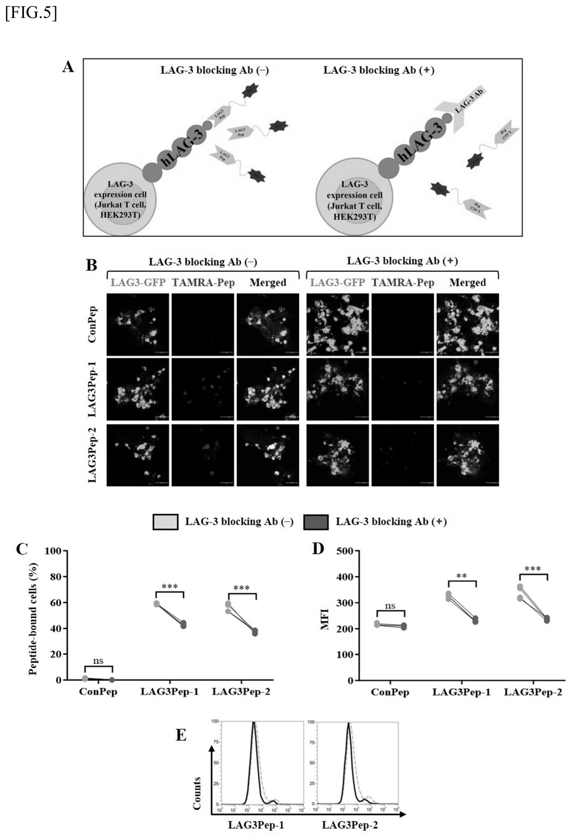

FIG. 5 shows results of analyzing the competitive binding between a LAG-3 binding peptide and an antibody. (a) A schematic diagram of analysis on the competitive binding between a LAG-3 blocking antibody and a LAG-3 binding peptide. (b) Results of observing a blockage of the binding of LAG3Pep-1 and LAG3Pep-2 in transfected HEK-293T cells by LAG-3 blocking antibody using immunofluorescence microscopy (Scale bar: 30 µm). (c-d) Results of measuring, via flow cytometry, a decrease in the binding of LAG3Pep-1 and LAG3Pep-2 due to competitive binding of LAG-3 blocking antibodies in a state in which Jurkat T cells were stimulated to express LAG-3, presented as a binding percentage and mean fluorescence intensity, respectively. *, P< 0.05; **, P< 0.01; ***, P< 0.001. (e) A result of flow cytometry for peptide binding, presented in a histogram.

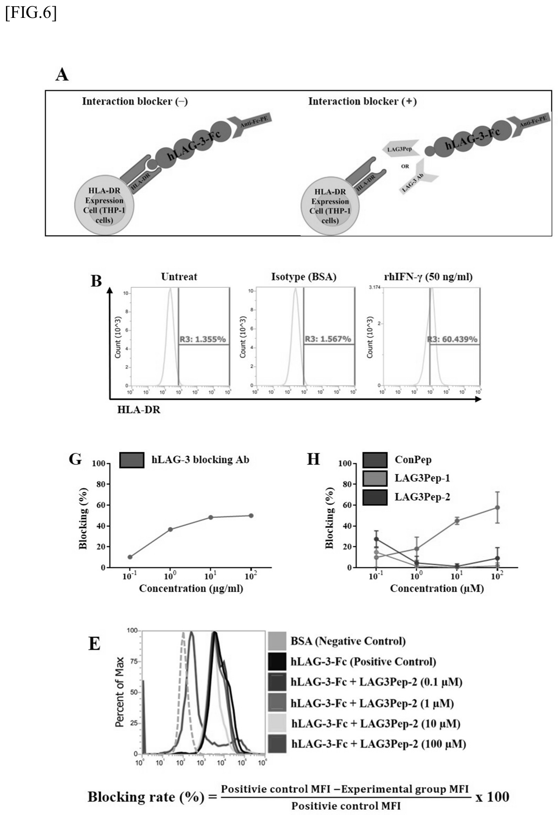

FIG. 6 shows a blocking effect of a LAG3 binding peptide on an interaction between LAG-3 and HLA-DR. (a) A schematic diagram of the cell-based blocking assay. (b) Results obtained via flow cytometry, presenting an expression level of HLA-DR in THP-1 human cells (an acute monocytic leukemia cell line) induced by recombinant human interferon-gamma for 48 hours. (c-d) Results obtained via flow cytometry, presenting a degree of blockage by LAG-3 blocking antibody and LAG3 binding peptide on an interaction between HLA-DR-expressing THP-1 cells and LAG-3 protein. It also shows a calculation formula for a degree of blockage. (e) A representative result of flow cytometry, presented in a histogram.

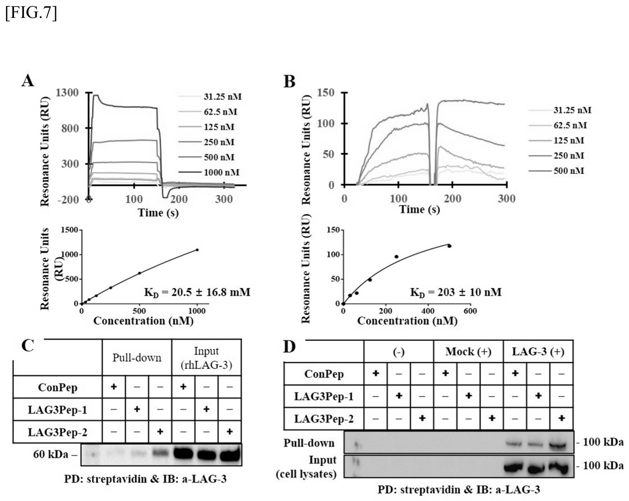

FIG. 7 shows results of analyzing a binding affinity and specific binding of a LAG3 binding peptide. Results identified via SPR analysis, showing a binding affinity for LAG3Pep-1 (a) and LAG3Pep-2 (b). Results of immunoblotting analysis using LAG-3 antibody after reacting a human LAG-3 recombinant protein (c) and cell lysates overexpressing human LAG-3 (d) with a biotin-peptide, followed by precipitation with streptavidin.

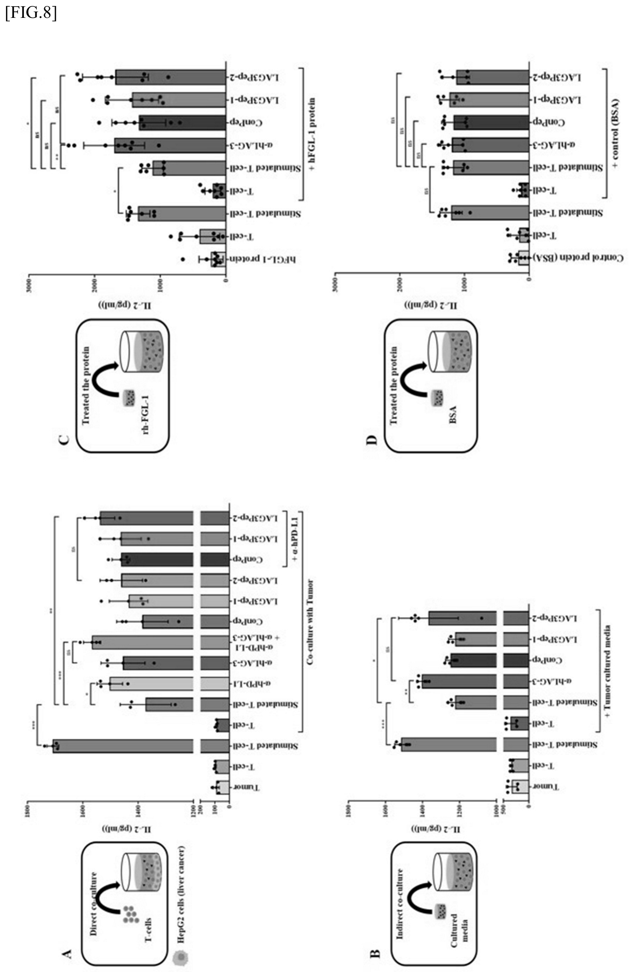

FIG. 8 shows IL-2 ELISA analysis as a functional test to observe recovery of T cells using LAG3Pep. Jurkat T cells were stimulated with PMA (50 ng/ml), ionomycin (1 µg/ml), and chloroquine (CQ) (100 µM) for 24 hours with blocking antibodies (LAG-3, PD-L1) and peptides (Control-pep, LAG3Pep-1, LAG3Pep-2). HepG2 cells were cultured for 24 hours to release FGL-1 proteins. After stimulation and culture, T cells were co-cultured with cancer cells, while unstimulated Jurkat T cells were used as a control group. A co-culture ratio is 1:5. (c) For mixed culture of culture media of Jurkat T cells and HepG2 cells, HepG2 cells were cultured for 48 hours to release FGL-1 proteins followed by mixed culture. (d) After culture and stimulation of Jurkat T cells with hFGL-1 protein (10 µg/ml) or isotype (BSA) (10 µg/ml), cells were co-cultured with hFGL-1 protein and BSA. As a control peptide, NSSSVDK peptides derived from phage surface proteins were used as a control group. *, P< 0.05; **, P< 0.01; ***, P< 0.001.

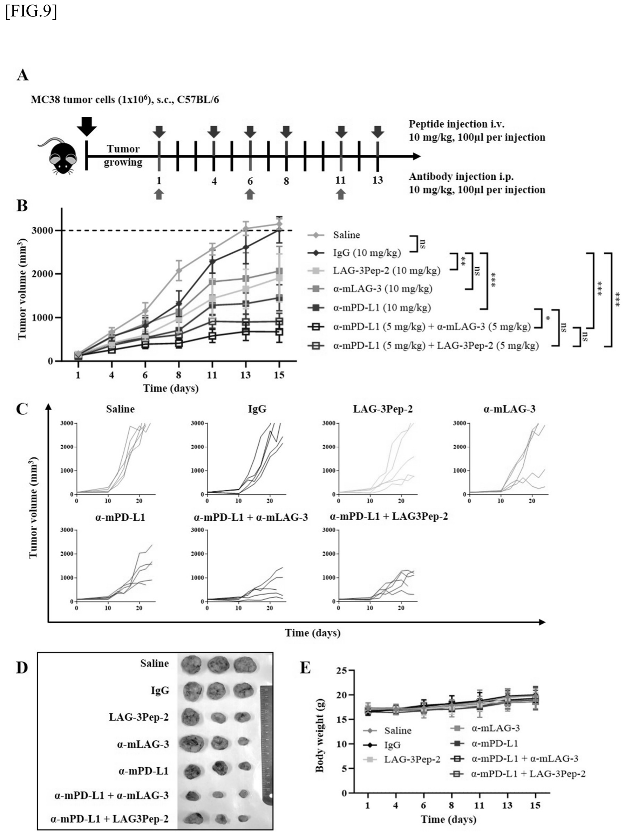

FIG. 9 shows results presenting that a LAG-3 binding peptide inhibits tumor growth when co-administered with a PD-L1 antibody in a model transplanted with a syngeneic MC38 mouse tumor. (a) A schematic diagram showing a peptide/antibody administration schedule in a MC38 tumor mouse model. (c) The difference in a tumor size between each group at the end of the 15-day experiment. Results of measuring a weight of mice (b) and a tumor size (d) every two days for 15 days. (e) A result showing a tumor size status of each mouse. n=6; Mean ± SEM, *, P < 0.05; **, P< 0.01; ***, P< 0.001.

### Best Mode for Carrying Out the Invention

The present disclosure provides a peptide that specifically binds to lymphocyte-activation gene-3 (LAG-3) consisting of an amino acid sequence represented by SEQ ID NO: 1 or SEQ ID NO: 2.

In the present disclosure, the amino acid sequence represented by SEQ ID NO: 1 is "CIRNDPAVC", referred to as "LAG3Pep-1" herein, and the amino acid sequence represented by SEQ ID NO: 2 is "CSVLNASGC", referred to as "LAG3Pep-2" herein.

The peptide of the present disclosure may be easily prepared by chemical synthesis known in the art. Representative methods include, but are not limited to, liquid or solid phase synthesis, fragment condensation, and F-MOC or T-BOC chemistry.

Additionally, the peptide of the present disclosure may be produced by genetic engineering methods. First, a DNA sequence encoding the peptide is synthesized according to a conventional method. DNA sequences may be synthesized by PCR amplification using appropriate primers. Alternatively, the DNA sequence may be synthesized by standard methods known in the art, for example, using an automated DNA synthesizer (e.g., those sold by Biosearch or AppliedBiosystems). The prepared DNA sequence is inserted into a vector containing one or more expression control sequences (e.g., promoter, enhancer, etc.) that are operatively linked to the DNA sequence to control expression of the DNA sequence, followed by transformation of a host cell with the recombinant expression vector formed therefrom. The resulting transformant is cultured under appropriate media and conditions to allow the DNA sequence to be expressed, and a substantially pure peptide encoded by the DNA sequence is recovered from the culture. The recovery may be performed using a method known in the art (e.g., chromatography). The term "substantially pure peptide" as used herein refers to a state in which the peptide according to the present disclosure does not substantially contain any other protein derived from the host.

In the present disclosure, a peptide consisting of an amino acid sequence represented by the SEQ ID NO: 1 or SEQ ID NO: 2 is a concept encompassing a functional variant thereof. The term "functional variant" as used herein refers to any similar sequence in which substitutions of some amino acid take place at amino acid positions that do not affect the property of the peptide of the present disclosure that specifically binds to LAG-3.

In addition, the present disclosure provides a polynucleotide encoding the peptide.

The term 'polynucleotide' as used herein refers to a polymer of deoxyribonucleotides or ribonucleotides existing in a single-stranded or double-stranded form. It encompasses RNA genomic sequences, DNA (gDNA and cDNA), and RNA sequences transcribed therefrom and includes analogs of natural polynucleotides unless otherwise specifically stated.

The polynucleotide includes not only a nucleotide sequence encoding the peptide, but also a sequence complementary to the sequence. The complementary sequence includes not only a perfectly complementary sequence but also a substantially complementary sequence.

Additionally, the polynucleotide may be modified. The modifications include additions, deletions, or non-conservative or conservative substitutions of nucleotides. The polynucleotide encoding the amino acid sequence is also interpreted to include a nucleotide sequence that shows substantial identity to the nucleotide sequence. The substantial identity may be a sequence that shows at least 80% homology, at least 90% homology, or at least 95% homology when the nucleotide sequence and any other sequence are aligned to the greatest extent possible and the aligned sequence is sequenced using an algorithm commonly used in the art.

In addition, the present disclosure provides a recombinant vector including the polynucleotide.

In addition, the present disclosure provides a transformant which is transformed with the recombinant vector and isolated.

As used herein, the term "vector" refers to a self-replicating DNA molecule used to carry a clonal gene (or other pieces of clonal DNA).

As used herein, the term "recombinant vector" refers to a plasmid, viral vector or other mediators known in the art capable of expressing an inserted nucleic acid in a host cell, and may be a polynucleotide encoding the peptide of the present disclosure that is operably linked to a conventional expression vector known in the art. The recombinant vector generally includes an origin of replication that may proliferate in a host cell, one or more expression control sequences that control expression (e.g., promoter, enhancer, etc.), a selective marker, and a polynucleotide encoding a peptide of the present disclosure that is operably linked to an expression control sequence. The transformant may be one that is transformed by the recombinant vector.

Preferably, the transformant may be obtained by introducing a recombinant vector including a polynucleotide encoding the peptide of the present disclosure into a host cell via methods known in the art, for example, but not limited to, transient transfection, microinjection, transduction, cell fusion, calcium phosphate precipitation, liposome-mediated transfection, DEAE dextran-mediated transfection, polybrene-mediated transfection, electroporation, gene gun, and other known methods for introducing nucleic acids into cells.

In addition, the present disclosure provides a drug delivery composition including the peptide as an active ingredient.

Preferably, the drug may be a peptide drug or an anticancer agent, and more preferably, the peptide drug may be a cytotoxic peptide having apoptosis or necrosis-inducing activity, but is not limited thereto.

The peptide according to the present disclosure may be used as an intelligent drug delivery that selectively delivers drugs to surrounding cancer cells through binding to T cells infiltrated within a cancer tissue. If the peptide of the present disclosure is used for cancer treatment by linking it with a conventionally known drug, the drug is selectively delivered to a cancer tissue by the peptide of the present disclosure, such that the efficacy of the drug may be increased, while the side effects of the drug on normal tissue may be significantly reduced.

The drug is an anticancer agent, and any anticancer agent that can be linked to the peptide of the present disclosure may be used without limitation as long as it is used for conventional treatment of cancer. Examples include gemcitabine, crizotinib, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, Adriamycin, methotrexate, busulfan, chlorambucil, cyclophosphamide, melphalan, nitrogen mustard, or nitrosourea. The linkage between the anticancer agent and the peptide of the present disclosure may be performed by methods known in the art, such as covalent bonding and cross-linking. To this end, the peptide of the present disclosure may be chemically modified, if necessary, within a range where its activity is not lost.

In addition, the present disclosure provides a pharmaceutical composition for preventing or treating a cancer, including the peptide as an active ingredient.

In addition, the present disclosure provides a pharmaceutical composition for preventing or treating a cancer, including the peptide and a PD-L1 antibody as active ingredients.

Preferably, the cancer may be, but is not limited to, colon cancer, breast cancer, lung cancer, pancreatic cancer, brain tumor, blood cancer, liver cancer, esophageal cancer, kidney cancer, rectal cancer, stomach cancer, thyroid cancer, bladder cancer, ovarian cancer, bile duct cancer, cholangiocarcinoma, gallbladder cancer, uterine cancer, cervical cancer, prostate cancer, head and neck cancer, laryngeal cancer, or oral cancer.

The pharmaceutical composition of the present disclosure may be prepared using pharmaceutically suitable and physiologically acceptable auxiliary agents in addition to the active ingredient, and solubilizers such as excipients, disintegrants, sweeteners, binders, coating agents, swelling agents, lubricants, glidants, or flavoring agents may be used as the auxiliary agents. The pharmaceutical composition of the present disclosure may be preferably formulated as a pharmaceutical composition by including one or more pharmaceutically acceptable carriers in addition to the effective ingredient for administration. In a composition formulated as a liquid solution, acceptable pharmaceutical carriers are those that are sterile and biocompatible, such as saline solution, sterile water, Ringer's solution, buffered saline, albumin injection solution, dextrose solution, maltodextrin solution, glycerol, ethanol, and mixtures of one or more of these components, and other conventional additives, such as antioxidants, buffers, and bacteriostatic agents, may be added as needed. In addition, diluents, dispersants, surfactants, binders, and lubricants may be additionally added to formulate the composition into injectable formulations such as aqueous solutions, suspensions, and emulsions, pills, capsules, granules, or tablets.

The pharmaceutical formulation form of the pharmaceutical composition of the present disclosure may be granules, powder, coated tablets, tablets, capsules, suppositories, syrups, juices, suspensions, emulsions, drops or injectable liquids, and sustained-release formulations of active compounds. The pharmaceutical composition of the present disclosure may be administered in a conventional manner via intravenous, intraarterial, intraperitoneal, intramuscular, intraarterial, intraperitoneal, intrasternal, transdermal, intranasal, inhalation, topical, rectal, oral, intraocular, or intradermal routes. The effective amount of the active ingredient of the pharmaceutical composition of the present disclosure refers to an amount required for the prevention or treatment of a disease. Therefore, it may be adjusted according to various factors including the type of disease, severity of the disease, type and content of the active ingredient and other ingredients contained in the composition, type of formulation, and the patient's age, weight, general health status, sex and diet, time of administration, route of administration, and secretion rate of the composition, treatment period, and concomitant drugs. Although not limited thereto, for example, in the case of adults, when the composition is administered once to several times a day, in the case of a compound, it may be administered at a dose of 0.1 ng/kg to 10 g/kg.

In addition, the present disclosure provides a composition for imaging T cells infiltrated into a cancer tissue, including the peptide as an active ingredient.

Preferably, the peptide may be labeled with, but is not limited to, a chromogenic enzyme, a radioisotope, a chromophore, a luminescent material, a fluorescer, a magnetic resonance imaging material, a superparamagnetic particle, or an ultrasuper paramagnetic particle.

For example, when a fluorescent substance is used as a detectable marker, the level of T cell infiltration in the cancer tissue may be diagnosed using fluorescence mediated tomography (FMT). That is, the peptide of the present disclosure labeled with a fluorescent substance may be circulated in the blood, and fluorescence caused by the peptide may be observed using fluorescence tomography. If strong fluorescence is observed, it is diagnosed that there is a high level of T cell infiltration within the cancer tissue.

Imaging of T cells infiltrating into a cancer tissue and diagnosis of the infiltration level may be used for purposes including, but not limited to, initial diagnosis of cancer, as well as monitoring of progression, treatment status, and response to therapeutic agents (especially anticancer immunotherapy). The peptide may be provided in a labeled state to facilitate identification, detection, and quantification of binding, as described above.

### Modes for Carrying Out the Invention

Hereinafter, the present disclosure will be described in detail according to Examples that do not limit the present disclosure. It should be noted that the following Examples of the present disclosure are intended only to illustrate the present disclosure and do not limit or restrict the scope of the present disclosure. Therefore, it is interpreted that what may be easily inferred by those skilled in the art to which the present disclosure pertains from the detailed description and Examples of the present disclosure falls within the scope of the present disclosure.

### <Experimental Example>

The following Experimental Examples are intended to provide Experimental Examples commonly applied to each Example according to the present disclosure.

### 1. Cell lines and reagents

HEK-293T (human embryonic kidney cells), Jurkat T, EOL, AML, and THP-1 (human leukemia cells), HepG2 and Hep3B (human hepatoma cells) were purchased from the American type culture collection (ATCC, Manassas, VA, USA). HEK-293T, HepG2, and Hep3B cells were cultured in DMEM low glucose (Gibco, Carslbad, CA, USA), and Jurkat T, EOL, AML, and THP-1 cells were cultured in RPMI-1640 (Gibco, Carslbad, CA, USA). All culture media were supplemented with 10% fetal bovine serum (Gibco), 100 U/ml penicillin, and 100 ug/ml streptomycin. All cells were cultured at 37°C using a CO₂ incubator.

Human recombinant LAG-3 and FGL-1 (Acro Biosystems), human recombinant IFN-γ (R&D), human LAG-3 antibody, FGL-1 antibody (Santa Cruz Biotechnology), and LAG-3 blocking antibody (AdipoGen) were purchased to be used. Human LAG-3 antibody, human HLA-DR antibody, anti-Fc antibody, human HLA-DR antibody, human IL-2 ELISA kit (Bio legend), human PD-L1 antibody, human PD-L1 blocking antibody, and T7-HRP antibody were purchased from ThermoFisher Scientific. Lipofectamine 3000 (Invitrogen), phorbol 12-myristate 13-acetate (PMA), chloroquine (CQ, Sigma-Aldrich), and ionomycin (Abcam) were purchased to be used, and the GFP-LAG-3 plasmid vector was purchased from Origene.

### 2. Western blotting analysis on LAG-3 protein expression in cells

HEK-293T cells (6.25×10⁵) were seeded in 6-well plates, transfected with human LAG-3 plasmid, and cultured for 48 hours. Non-transfected cells were used as a control group. The next day, cells were harvested and lysed using mammalian protein extraction reagent (M-PER) and a protease inhibitor mixture to harvest proteins and applied to Western blotting using human LAG-3 antibody and HRP secondary antibody.

### 3. Analysis on LAG-3 protein expression in cells using confocal microscopy and flow cytometry

HEK-293T cells (1.25×10⁵) were seeded in 4-well chamber slides, transfected with human LAG-3 plasmid, and cultured for 48 hours. The next day, cells were washed twice with phosphate buffer and reacted with 1% BSA at room temperature for 1 hour to reduce nonspecific binding of antibodies. Afterwards, the cells were reacted with LAG-3 antibody at room temperature for 1 hour, and then reacted with secondary antibody (Alexa594) at room temperature for 1 hour. After fixation, cells were washed, counterstained with 4',6-diamidino-2-phenylindole (DAPI), treated with an anti-fade reagent to preserve fluorescence, and analyzed by confocal microscopy. Non-transfected cells were used as a control group.

In the same way, using Jurkat T cells stimulated with phorbol 12-myristate 13-acetate (PMA, 50 ng/ml), ionomycin (1 µg/ml), and chloroquine (CQ, 100 µM) as well as unstimulated Jurkat T cells, 10⁶ cells were harvested in consideration that they are floating cells, and staining was performed in an e-tube, some of which were smeared on a slide in the last step, covered up with a cover slide, and then analyzed using a confocal microscope (NanoScope, Daejeon).

In the case of Jurkat T cells, to determine the level of LAG-3 expression according to the treatment method of PMA/Iono/CQ, 10⁶ Jurkat T cells treated with drugs in various ways were harvested, washed twice with phosphate buffer, and reacted with 1% BSA at room temperature for 30 minutes to reduce nonspecific binding of antibodies. Afterwards, a reaction was performed with LAG-3 antibody labeled with green fluorescein isothiocyanate (FITC) at 4°C for 1 hour. After washing several times, 300 µl of cell suspension suspended in phosphate buffer was analyzed using Attune NxT flow cytometry (Thermo Fisher Scientific, Waltham, USA). Unstimulated Jurkat T cells were used as a control group.

### 4. Bio-panning using the T7 phage peptide library

The T7 phage peptide library used was constructed using a phage vector purchased from Novagen. Hek-293T cells were cultured in 35 mm dishes and transfected with LAG-3 plasmid when reaching 70-80% confluency. The cells were washed twice with phosphate buffer for 5 minutes each. For the subtraction procedure, 1×10⁹ plaque-forming units (pfu) of phage library were cultured in Hek-293T cells at 4°C for 1 hour. The phages unbound to the cells were recovered and cultured with LAG-3-transfected Hek-293T cells at 4°C for 1 hour. Afterwards, the unbound phages were washed with phosphate buffer containing 10 mg/ml bovine serum albumin (BSA). Phages bound to cells were eluted with BL21 (OD: 1.0) culture medium at room temperature for 10 minutes. The eluate was used for titration, and the remaining elution phage clone was placed in 10 ml BL21 (OD: 0.5) to be subjected to a reaction for 3-4 hours until the T7 phage underwent a lysis cycle to be lysed transparently. The process was repeated a total of 4 times, and then the dilution was continued. The lysate was inoculated into LB medium Petri plates and cultured overnight at 37°C. The phage titer was measured by counting the number of colonies.

### 5. Sequencing and peptide synthesis

After five rounds of bio-panning, the inserted clones were detected by polymerase chain reaction (PCR). Each of the 80 clones with DNA inserts was sequenced (Macrogen, Seoul, South Korea). Sequence similarity was analyzed using the Clustal W program. As LAG-3 binding peptides, LAG3Pep-1 (CIRNDPAVC; SEQ ID NO: 1) and LAG3Pep-2 (CSVLNASGC; SEQ ID NO: 2) were synthesized (Peptron Inc. Daejeon, Korea). Fluorescein isothiocyanate (FITC) and biotin were conjugated to the N-terminus of each peptide. Lyophilized peptides were reconstituted in distilled water or dimethyl sulfoxide (DMSO) to a concentration of 50 mM or 10 mM, respectively. Afterwards, it was further diluted to a concentration of 10 µM with phosphate buffer. NSSSVDK, a peptide sequence present in phage coat protein, was used as a control peptide.

### 6. Phage binding ELISA assay

Hek-293T cells (2.5×10⁴) were seeded in a 96-well plate and transfected with LAG-3 plasmid. Culture was performed for 48 hours. Non-transfected Hek-293T cells were used as a control group. Plates were blocked with 10 mg/ml BSA for 1 hour at room temperature. After washing the wells six times with Tris-buffered saline-Tween 20 (0.1%), 100 µl of phage clones (1×10⁹ pfu/well) were added and reacted at 4°C for 1 hour. After washing, horse-radish peroxidase (HRP)-conjugated T7 tail fiber antibody (diluted 1:10,000 in blocking buffer) was added and reacted at room temperature for 1 hour. After washing the plate six more times, a substrate consisting of 3,3',5,5'-tetramethylbenzidine was added and reacted at room temperature for 10-15 minutes. The reaction was finally stopped by adding 100 µl of 2 M H₂SO₄ (stop solution), and the plates were analyzed at 450 nm with a microplate reader.

### 7. Analysis on the LAG-3 peptide binding via immunofluorescence and flow cytometry

Hek-293T cells (1.25×10⁵) were seeded in 4-well chamber slides, transfected with human LAG-3 plasmid, and cultured for 48 hours. Non-transfected cells were used as a control group. The next day, cells were washed twice with phosphate buffer and reacted with 1% BSA at room temperature for 1 hour to reduce nonspecific binding of peptides. Afterwards, cells were reacted with TAMRA-conjugated peptide at a concentration of 25 µM at 4°C for 1 hour. After fixation, cells were washed, counterstained with DAPI, treated with an anti-fade reagent to preserve fluorescence, and analyzed using a confocal microscope (Nanoscope System).

For flow cytometry, Jurkat T cells (1×10⁶) stimulated with PMA (50 ng/ml), ionomycin (1 µg/ml), and CQ (100 µM) were harvested and suspended in culture medium containing 1% BSA for blocking at room temperature for 1 hour. Afterwards, the cells were further reacted with fluorescein isothiocyanate-labeled LAG-3 binding peptide (10 µM) at 4°C for 1 hour. After washing several times, 300 µl of cell suspension suspended in phosphate buffer was applied to Attune NxT flow cytometry (Thermo Fisher Scientific).

### 8. Competitive binding strength of LAG-3 binding peptides

Hek-293T cells (1.25×10⁵) were seeded in 4-well chamber slides and transfected with human LAG-3 plasmid, and the next day, the cells were washed twice with phosphate buffer and reacted with 1% BSA at room temperature for 1 hour to reduce nonspecific binding. Afterwards, cells were blocked with human LAG-3 blocking antibody (10 µg/ml) at 4°C for 30 minutes, and then reacted with TAMRA-conjugated peptide at a concentration of 25 µM at 4°C for 1 hour. After fixation, cells were washed, counterstained with DAPI, treated with an anti-fade reagent to preserve fluorescence, and analyzed using a confocal microscope (Nanoscope System).

Similarly, for flow cytometry, Jurkat T cells (1×10⁶) stimulated with PMA (50 ng/ml), ionomycin (1 µg/ml), and CQ (100 µM) were harvested and suspended in culture medium containing 1% BSA for 1 hour at room temperature to block nonspecific binding. Afterwards, cells were blocked with human LAG-3 blocking antibody (10 µg/ml) at 4°C for 30 minutes and then reacted with fluorescein isothiocyanate-conjugated peptide at a concentration of 10 µM at 4°C for 1 hour. After washing several times, 300 µl of cell suspension suspended in phosphate buffer was applied to Attune NxT flow cytometry (Thermo Fisher Scientific). A group that did not use LAG-3 blocking antibody was used as a control group.

### 9. Inhibition intensity of LAG-3 binding peptides on the LAG-3/HLA-DR interaction

To increase expression of HLA-DR (MHC class II) in THP-1 cells of blood cancer cells, human recombinant IFN-γ was treated, and after culturing for 48 hours to express HLA-DR on the cell membrane, the cells were harvested and treated with LAG-3 binding peptide and LAG-3 blocking antibody at various concentrations before treatment of human recombinant LAG-3 protein, followed by a reaction at 4°C for 30 minutes to coat LAG-3 protein. The coated LAG-3 protein was added to the cells and reacted at 4°C for 30 minutes, unbound proteins were washed off, and the proteins were stained using antibodies against Fc. After washing several times, 300 µl of cell suspension suspended in phosphate buffer was applied to Attune NxT flow cytometry (Thermo Fisher Scientific). BSA was used as a control group.

### 10. Analysis on the binding specificity of LAG-3 binding peptides

The binding specificity of the LAG-3 binding peptide was measured by pull-down analysis. After pull-down with LAG-3 binding peptide using human recombinant LAG-3 protein, it was applied to immunoblotting using LAG-3 antibody. In the same manner, cell lysates with LAG-3 overexpressed were knocked down using human LAG-3 plasmid DNA and then subjected to immunoblotting using LAG-3 antibodies.

### 11. Measurement of KD values of LAG-3 binding peptides

Using surface plasmon resonance (SPR), affinity constants (K_{D}) and kinetics (Kₐ and K_{d}) were measured. LAG-3 binding peptide (15 µM) was immobilized on the surface of a streptavidin (SA) chip, and 1x phosphate buffer (PH 7.4) was flowed at 25 µl/min for 10 minutes. Afterwards, the remaining unbound proteins were removed by flowing 2 M NaCl at 50 µl/min for 5 minutes. LAG-3 protein was dissolved in working buffer and flowed at six different concentrations (31.25 nM, 62.5 nM, 125 nM, 250 nM, 500 nM, 1000 nM). Each concentration was injected and flowed at 50 µl/min for 2 minutes and 30 seconds. Each RU was recorded. Calculations were performed using Scrubber and clamp software.

### 12. T-cell activation assay

Jurkat T cells (1×10⁶) were inoculated into a T25 flask and stimulated for 24 hours using PMA (50 ng/ml), ionomycin (1 µg/ml), and CQ (100 µM). HepG2 cells (5×10³) were inoculated in a 96-well plate to induce secretion of FGL-1 for 48 hours. To co-culture the two types of cells, stimulated Jurkat T cells (2.5×10⁴) were mixed with HepG2 at a ratio of 1:5. Similarly, for indirect co-culture, stimulated Jurkat T cells (2.5×10⁴) were mixed with HepG2 cell culture medium. Finally, to observe the direct effect, stimulated Jurkat T cells (2.5×10⁴) were treated with recombinant FGL-1 (10 µg/ml) protein. In this process, LAG-3 binding peptide (50 µM), human LAG-3 antibody (10 µg/ml), human PD-L1 antibody (10 µg/ml), PD-L1/LAG-3 antibody, and LAG-3 binding peptide + human PD-L1 antibody were added. Afterwards, culture was carried out for an additional 24 hours for reaction. Control peptides were also used for comparison. After each culture time, the supernatant was harvested, and IL-2 secretion was measured using an ELISA kit for measuring human IL-2.

### 13. In-vivo antitumor therapy

6-8-week-old C57BL/6 mice were purchased. Mice were bred and kept under the guidelines of the Institutional Animal Care and Use Committee (IACUC) of Kyungpook National University (2023-0184). Tumors were injected into the lower right flank of mice. For in-vivo antitumor treatment, mice were randomly selected and grouped when tumor size reached approximately 100-150 mm³. Thereafter, the peptide was injected intravenously three times a week for two weeks at a dose of 10 mg/kg, and similarly the antibody was injected intraperitoneally three times at the same dose. Tumor size was measured using a digital caliper, and tumor volume was calculated using the formula (V = length × width² × 0.52) (where length (L) is the longest dimension, and width (W) is the shortest dimension, parallel to the mouse body). All mice were checked for ulcer severity after treatment, treatment was terminated when the tumor size reached approximately 3000 mm³, and the mice were sacrificed.

### 14. Statistical analysis

Statistical significance was measured by the unpaired Student t-test in comparison of two groups, and one- or two-way ANOVA was applied in the comparison of multiple groups. Values are presented as mean ± SD for three independent samples. If the P value is <0.05, it was considered statistically significant.

### <Example 1> Construction and analysis of cell lines overexpressing the LAG-3 protein

To find peptides capable of selectively binding to LAG-3-expressing cells or blocking LAG-3, a T7 phage library of random peptides was screened against LAG-3-overexpressing cells. To prepare LAG-3-overexpressing cells, LAG-3 plasmid DNA linked to co-express green fluorescent protein (GFP) was transiently transfected into Hek-293T cells and cultured for 48 hours.

As a result, after transfection, expression of GFP and LAG-3 was high at 48 hours, which was detected via microscopic analysis (FIG. 1a) and Western blotting (FIG. 1b). In subsequent experiments, cells reached 48 hours after transfection were used. To further observe LAG-3 expression, LAG-3-transfected Hek-293T cells were stained with LAG-3 antibody and examined under an immunofluorescence microscope. Non-transfected cells were used as a control group, and more LAG-3 antibodies were found to be stained in LAG-3-transfected cells (FIG. 1c). Additionally, observation was made on whether expression of LAG-3 and GFP was mainly present on the cell membrane (FIG. 1c).

### <Example 2> Analysis of LAG-3 expression in Jurkat T cells

Jurkat T cells were stimulated with PMA (50 ng/ml), ionomycin (1 µg/ml), and CQ (100 µM) by various combination treatments at various times to detect the level of LAG-3 expression on the cell membrane via flow cytometry. Unstimulated Jurkat T cells were used as a control group.

As a result, when PMA (50 ng/ml), ionomycin (1 µg/ml), and CQ (100 µM) were treated together and cultured for 48 hours, the percentage of cells expressing the highest level of LAG-3 (FIGS. 2a and 2c) and mean fluorescence intensity (MFI) was observed (FIG. 2b). In the subsequent studies, Jurkat T cells reached 48 hours after stimulation were used. When expression level of LAG-3 on the cell membrane was observed using immunofluorescence microscopy by treating other blood cancer cell lines, EOL and AML, with PMA (50 ng/ml), ionomycin (1 µg/ml), and CQ (100 µM), a higher level of LAG-3 expression was observed in Jurkat T cells compared to other blood cancer cell lines (FIG. 2d).

### <Example 3> Bio-panning of LAG-3 binding peptides using the T7 phage library

As a result of performing bio-panning against LAG-3-overexpressing cells five times using a T7 phage library with random sequence peptides, the phage titer was approximately 40-fold higher in the third round compared to the first round, and the titer decreased in the fourth round before increasing again in the fifth round (FIG. 3a). A total of 80 phage clones were randomly selected from the phage clones that emerged in the third round. To identify the sequence of the inserted clones, polymerase chain reaction and DNA sequencing were conducted on each selected clone. As a result of determining using protein BLAST database based on the analyzed sequence, a peptide (CIRNDPAVC: SEQ ID NO: 1, named LAG3Pep-1) with a sequence similar to the HLA-DR sequence, a cell surface receptor of MHC class II that interacts with LAG-3, was selected and synthesized (FIG. 3c). Additionally, 13 phage clones with a length of 7 amino acids or more were selected after excluding sequences appearing in untransfected cells among the transformed cells (FIG. 3c). The cell binding of 13 selected phage clones was examined by phage binding ELISA targeting transfected or non-transfected Hek-293T cells, and the peptide of clone 3 (CSVLNASGC: SEQ ID NO: 2, designated LAG3Pep-2) showing the highest level of binding was selected and synthesized (FIGS. 3b and 3d).

### <Example 4> Analysis on the binding of LAG-3 binding peptide to LAG-3 expressing cells

To determine the degree of binding of the peptide to LAG-3, a peptide labeled with a red fluorescent reagent was synthesized and treated in Hek-293T cells transfected with LAG-3 followed by observation under an immunofluorescence microscope. Non-transfected cells were used as a control group. Meanwhile, Jurkat T cells stimulated with PMA, ionomycin, and CQ to increase expression of LAG-3 were treated with the peptide, and binding was observed using a flow cytometer. Unstimulated Jurakt T cells were used as a control group.

As a result, LAG-3Pep-1 and LAG-3Pep-2 peptides (red fluorescence) were found to bind more strongly in Hek-293 cells with higher expression of LAG-3 (green fluorescence) compared to control cells (FIG. 4a). Additionally, more LAG-3Pep-1 and LAG-3Pep-2 peptides were bound in stimulated Jurkat T cells compared to control cells (FIGS. 4b and 4c).

Next, observation was made on whether blocking LAG-3 using a LAG-3 blocking antibody reduced the binding of the LAG-3 binding peptide (FIG. 5a). First, using Hek-293T cells transfected with LAG-3, LAG-3 blocking antibody was treated first, then LAG-3Pep-1 and LAG-3Pep-2 peptides (red fluorescence) were treated, and, as a result of detecting with immunofluorescence microscopy, it was revealed that the binding strength of the LAG-3 binding peptide was reduced in the group treated with LAG-3 blocking antibody compared to the group without treatment (FIG. 5b). Similarly, when Jurakt T cells stimulated with PMA, ionomycin, and CQ were treated first with LAG-3 blocking antibody and then with LAG-3 binding peptides, followed by observation via flow cytometry, it was determined that the binding of LAG-3Pep-1 and LAG-3Pep-2 peptides was reduced in the group treated with LAG-3 blocking antibody compared to the untreated control group (FIGS. 5c-e).

### <Example 5> Inhibitory effect of LAG-3 binding peptide on the interaction between HLA-DR and LAG-3

It was attempted to determine whether the binding or interaction between LAG-3-Fc and HLA-DR was affected when THP-1 cells expressing HLA-DR, a ligand of LAG-3, were reacted with recombinant LAG-3-Fc protein and then treated with LAG-3 blocking antibody or LAG-3 binding peptide (FIG. 6a).

First, to increase the HLA-DR expression level in THP-1 cells, human recombinant IFN-γ was treated and cultured for 48 hours, and then the HLA-DR expression level was detected by flow cytometry using HLA-DR antibody. Cells not treated with human recombinant IFN-γ and cells treated with BSA were used as control groups. As a result, the highest HLA-DR expression was observed in cells treated with human recombinant IFN-γ (FIG. 6b). When treating cells with recombinant LAG-3-Fc protein, the LAG-3 binding peptide was treated at various concentrations, and an amount of LAG-3-Fc protein bound to HLA-DR on the THP-1 cell membrane was stained with an antibody against Fc, followed by measurement using a flow cytometer. LAG-3 blocking antibody was used as a positive control group, and a control peptide with the NSSSVDK sequence was used as a negative control group. As a result, the LAG-3 blocking antibody showed an increasing blocking ratio of the LAG-3/HLA-DR interaction as the concentration increased (FIG. 6c), and the LAG-3 binding peptide showed an increasing blocking ratio of the LAG-3/HLA-DR interaction as the concentration increased only for LAG3Pep-2 (FIGS. 6d and 6e). The formula for a blocking ratio was calculated using values of the mean fluorescence intensity from flow cytometry.

### <Example 6> Affinity analysis of LAG-3 binding peptides for recombinant LAG-3 protein

To measure the binding affinity (K_{D}) value of the peptide, SPR analysis was performed by coating a biotin-peptide on a streptavidin chip and flowing the recombinant LAG-3 protein.

As a result, the affinity of LAG3Pep-1 for LAG-3 protein was approximately 20.49 ± 16.79 µM (FIG. 7a), and the affinity of LAG3Pep-2 was approximately 0.203 ± 0.01 µM (FIG. 7b). In addition, after reacting the recombinant LAG-3 protein and biotin-labeled peptides, pull-down with streptavidin beads and immunoblotting with LAG-3 antibodies resulted in more detection of LAG-3 protein by LAG3-binding peptides, especially by LAG3Pep-2, compared to the control group (FIG. 7c). After reacting the cell lysate overexpressing LAG-3 with biotin-labeled peptides, as a result of performing pull-down and immunoblotting as described above, more LAG-3 proteins were detected by LAG3-binding peptides, especially by LAG3Pep-2, compared to the control group (FIG. 7d).

### <Example 7> IL-2 production by T cells following co-culture of HepG2 liver cancer cells and treatment with recombinant FGL-1 and the effect of LAG-3 binding peptide therefor.

Investigation was made on whether the interaction between FGL-1 secreted from HepG2 hepatoma cells and LAG-3 of T cells inhibits T cell activity and, if so, whether it is blocked by a LAG-3 binding peptide. For this purpose, in the first experiment, Jurkat T cells were cultured with stimulation for 24 hours using PMA, ionomycin, and CQ. HepG2 cells were cultured in 96-well plates for 48 hours for FGL-1 secretion. Subsequently, Jutakt T cells and HepG2 cells were co-cultured at a ratio of 5:1 for 24 hours and co-treated with LAG-3 blocking antibody and PD-L1 blocking antibody/LAG-3 binding peptide (FIG. 8a). In the second experiment, only the cell culture fluid of HepG2 cells was collected and then treated to stimulated Jurkat T cells. At this time, PD-L1 blocking antibody was not used, and only LAG-3 blocking antibody and LAG-3 binding peptide were treated, followed by culture for 24 hours (FIG. 8b). In the third experiment, stimulated Jurkat T cells were directly treated with recombinant FGL-1 protein, and then LAG-3 blocking antibody and LAG-3 binding peptide were treated along, followed by culture for 24 hours (FIGS. 8c-d). As a control group, BSA-treated group and NSSSVDK peptide were used. After completion of co-culture, the cell culture was harvested, and the IL-2 secretion level in the culture medium was identified using a human IL-2 ELISA kit.

As a result, under the three experimental conditions mentioned above, which are co-culture of HepG2 cells, treatment with HepG2 cell culture medium, and treatment with recombinant FGL-1, T cells were activated, leading to reduction in IL-2, a cytokine produced thereby (FIGS. 8a-c). In contrast, co-treatment with LAG-3 blocking antibody or LAG3Pep-2 under the conditions significantly increased IL-2 secretion again (FIGS. 8a-c).

### <Example 8> Anticancer therapeutic effect of LAG-3 binding peptide in mouse tumor models

To identify the anticancer effect in a syngeneic mouse tumor model, LAG-3 binding peptide was administered alone or in combination with PD-L1 antibody to mice in which tumors were induced using MC38 mouse colon cancer cells (FIG. 9a). At this time, the LAG-3 binding peptide was administered intravenously to the mice every other day for a total of 6 times, and the PD-L1 antibody was administered intraperitoneally to the mice every 5 days for a total of 3 times.

As a result, compared to the control group and phosphate buffer group, cancer growth was partially inhibited by administration of LAG3Pep-2 and PD-L1 antibodies alone, and tumor growth was inhibited by co-administration of LAG3Pep-2 and PD-L1 antibodies compared to the single administered group (FIGS. 9b-d). During the experimental period, the body weight of the animals was similar in all experimental groups without significant fluctuations (FIG. 9e).

While a specific part of the present disclosure has been described in detail above, it is clear for those skilled in the art that this specific description is merely preferred examples, and the scope of the present disclosure is not limited thereby. Accordingly, the substantial scope of the present disclosure will be defined by the appended claims and their equivalents.

## Claims

1. A peptide specifically binding to lymphocyte-activation gene-3 (LAG-3), consisting of an amino acid sequence represented by SEQ ID NO: 1 or SEQ ID NO: 2.

2. A polynucleotide encoding the peptide according to claim 1.

3. A recombinant vector comprising the polynucleotide according to claim 2.

4. A transformant which is transformed with the recombinant vector according to claim 3 and isolated.

5. A drug delivery composition comprising the peptide according to claim 1 as an active ingredient.

6. The drug delivery composition of claim 5, wherein the drug is a peptide drug or an anticancer agent.

7. The drug delivery composition of claim 6, wherein the peptide drug is a cytotoxic peptide having an apoptosis or necrosis-inducing activity.

8. The drug delivery composition of claim 6, wherein the anticancer agent is any one or more selected from the group consisting of gemcitabine, crizotinib, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, Adriamycin, methotrexate, busulfan, chlorambucil, cyclophosphamide, melphalan, nitrogen mustard, and nitrosourea.

9. A pharmaceutical composition for preventing or treating a cancer, comprising the peptide according to claim 1 as an active ingredient.

10. A pharmaceutical composition for preventing or treating a cancer, comprising the peptide according to claim 1 and a PD-L1 antibody as active ingredients.

11. The pharmaceutical composition of claim 9 or 10, wherein the cancer is any one or more selected from the group consisting of colon cancer, breast cancer, lung cancer, pancreatic cancer, brain tumor, blood cancer, liver cancer, esophageal cancer, kidney cancer, rectal cancer, stomach cancer, thyroid cancer, bladder cancer, ovarian cancer, bile duct cancer, cholangiocarcinoma, gallbladder cancer, uterine cancer, cervical cancer, prostate cancer, head and neck cancer, laryngeal cancer, and oral cancer.

12. A composition for imaging T cells infiltrated into a cancer tissue, comprising the peptide according to claim 1 as an active ingredient.

13. The composition of claim 12, wherein the peptide is labeled with any one selected from the group consisting of a chromogenic enzyme, a radioisotope, a chromophore, a luminescent material, a fluorescer, a magnetic resonance imaging material, a superparamagnetic particle, and an ultrasuper paramagnetic particle.
